# EUROPÄISCHE PATENTSCHRIFT

(11) **EP 0 691 970 B1**
(45) Veröffentlichungstag und Bekanntmachung des Hinweises auf die Patenterteilung: **11.11.1998**
(21) Anmeldenummer: 94911932.5
(22) Anmeldetag: 19.03.1994
(51) Int. Cl.: C07D 487/04, A61K 31/495

(54) **IMIDAZOLOCHINOXALINONDERIVATE ALS EAA ANTAGONISTEN**
IMIDAZOLOQUINOXALINONE DERIVATIVES AS EAA ANTAGONISTS
DERIVES D'IMIDAZOLO-QUINOXALINONE UTILISES COMME ANTAGONISTES D'AMINOACIDES A EFFET EXCITATIF

(30) Priorität: 31.03.1993 DE 4310521; 04.09.1993 DE 4329970
(43) Veröffentlichungstag der Anmeldung: 17.01.1996
(73) Patentinhaber: BASF AKTIENGESELLSCHAFT, 67056 Ludwigshafen (DE)
(72) Erfinder: TREIBER, Hans-Joerg, D-68782 Bruehl (DE); BEHL, Berthold, D-67117 Limburgerhof (DE); HOFMANN, Hans, Peter, D-67117 Limburgerhof (DE)
(86) Internationale Anmeldenummer: EP9400871
(87) Internationale Veröffentlichungsnummer: WO9422865

(56) Entgegenhaltungen:
- EP-A- 0 518 530
- WO-A-93/04066
- GB-A- 2 043 064

## Beschreibung

Die vorliegende Erfindung betrifft neue Imidazolo-chinoxalinone, Verfahren zu deren Herstellung, sowie deren Verwendung zur Bekämpfung von Krankheiten.

In den DE-OS 3 004 750 und DE-OS 3 004 751 werden eine Reihe von Imidazolo-chinoxalinonen Substanzen beschrieben, die antiallergische Wirkungen besitzen. Ferner sind substituierte Imidazolo-chinoxalinone als Phosphodiesterasehemmer bzw. Herz-Kreislaufbeeinflussende Mittel in US 5 166 344 (= EP 400 583) beansprucht.

Auf dem ZNS-Sektor sind in US 5 163 196 (= EP 518 530) verschiedene Heterocyclen, darunter auch einige Imidazolo-chinoxalinone genannt, die eine Wirkung als Antagonisten der excitatorisch wirkenden Aminosäuren (EAA-Antagonisten) entfalten. Ferner sind in der US 5 182 386 Imidazolo-chinoxaline beschrieben, die Antagonisten oder inverse Agonisten des GABA-Rezeptors darstellen und zur Bekämpfung von Angstzuständen, Schlafstörungen, Krampfzustanden sowie zur Verbesserung des Gedachtnisses dienen können.

Es wurde nun gefunden, daß neue Imidazolo[1,2-a]chinoxalinone der Formel I, 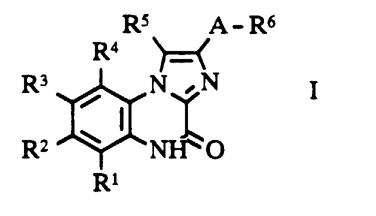 worin
- A: eine gesättigte oder ungesättigte Alkylengruppe mit 1-5 C-Atomen oder eine Bindung ist,
- R⁶: eine Formylgruppe oder eine Carboxylgruppe, die in Form ihres Salzes mit einem physiologisch verträglichen Amin-oder Metallkation vorliegen kann, den Rest COOR⁷, wobei R⁷ einen C₁-C₈-Alkylrest, eine Cycloalkylgruppe mit 3-8 C-Atomen im Ring, einen Benzylrest, einen der Reste -(CH₂)ₙ-OR⁸, in denen n für die Zahl 2-4 und R₈ für eine C₁-C₃-Alkylgruppe stehen, eine C₁-C₄-Hydroxyalkyl-, C₁-C₄-Alkylcarbonyl-, Nitrilo-, Tetrazolyl-, Carbonylaminotetrazol-, eine Aldoxim-, eine C₁-C₃-Alkoxyaldoxim-, oder eine gegebenenfalls substituierte Carbamoylgruppe darstellt, bedeutet und
- R²: ein Fluor-, Chlor- oder Bromatom, eine Trifluormethyl-, Trifluormethoxy-, Cyano-, Nitro-, Amino-, C₁-C₅-Alkyl-, C₁-C₅-Alkoxy-, Mono- oder Dialkylamino-, C₁-C₆-Alkylthio-, C₁-C₆-Alkylsulfinyl-, C₁-C₆-Alkylsulfonyl-, Aminosulfonyl-, Di-C₁-C₆-alkylaminosulfonyl- oder C₁-C₄-Alkoxycarbonylgruppe bedeutet,
- R¹, R³, R⁴: die gleich oder verschieden sind und Wasserstoff-, Fluor-, Chlor- oder Bromatome, Trifluormethyl-, Trifluormethoxy-, Cyano-, Nitro-, Amino-, C₁-C₅-Alkyl-, C₁-C₅-Alkoxy-, Mono- oder Dialkylamino-, C₁-C₆-Alkylthio-, C₁-C₆-Alkylsulfinyl -, C₁-C₆-Alkylsulfonyl-, Aminosulfonyl-, Di-C₁-C₆-alkylaminosulfonyl- oder C₁-C₄-Alkoxycarbonylgruppen bedeuten, oder eines der Paare R¹, R²; R²,R³ oder R³,R⁴ auch die Gruppe -(CH₂)₄- oder -CH=CH-CH=CH- ist
- R⁵: ein Wasserstoffatom, eine C₁-C₅-Alkylgruppe oder eine gegebenenfalls durch Chlor, Fluor, Trifluormethyl oder C₁₋₄-Alkyl substituierte Phenylgruppe bedeutet,
wobei jedoch nicht gleichzeitig
- A: eine Bindung,
- R⁶: eine Formyl-, Ethoxycarbonyl-, Hydroxymethyl-, Tetrazolyl-, Carbonsäureamid- oder 5-Carbonylaminotetrazolylgruppe,
- R¹, R⁴, R⁵: Wasserstoffatome und
- R² und R³: identisch und Chlor- oder Bromatome in der 7- und 8-Stellung sein können.
neue, hochwirksame Antagonisten der sogenannten excitatorisch wirksamen Aminosäuren (EAA-Antagonisten) darstellen. Sie sind daher besonders zur Therapie von neurologischen Störungen geeignet.

Bevorzugte Verbindungen sind solche, die im Benzolring als Substituenten R² - R⁴ eine oder zwei elektronenziehende Substituenten, wie Halogenatome, Nitro- oder Trifluormethylgruppen tragen.

Im Imidazolring ist die bevorzugte Substitution in R⁵ durch eine Methyl-, Ethyl- oder Phenylgruppe gegeben, wogegen R⁶ vorzugsweise eine direkt mit dem Ring oder mit einer Ethylenkette verknüpfte (A = Bindung) Carbonsäure- oder Carbonestergruppe darstellt.

Speziell seien solche Verbindungen der Formel I genannt, worin R¹ die in Anspruch 1 angegebene Bedeutung besitzt, worin R² ein Chlor- oder Bromatom, eine Trifluormethyl-, Trifluormethoxy-, oder Nitrogruppe bedeutet; R³ ein Wasserstoff- oder Chloratom oder eine Nitrogruppe ist, R⁴ ein Wasserstoff- oder Chloratom darstellt; R⁵ eine Methyl-, Ethyl- oder Phenylgruppe ist; A eine Bindung, eine Vinyl- oder Ethylengruppe darstellt und R₆ eine Carbonsäure- oder Estergruppe bedeutet.

Die Carbonsäuren können in üblicher Weise in ihre Metallsalze oder mit Ammoniak oder geeigneten organischen Stickstoffbasen in physiologisch verträgliche Ammoniumsalze überführt werden.

Als besonders bevorzugt sind folgende Verbindungen zu nennen:
a. 4,5-Dihydro-7-chlor-1-methyl-4-oxo-imidazolo[1,2-a]chinoxalin-2-carbonsäure
b. 4,5-Dihydro-1-methyl-7-nitro-4-oxo-imidazolo[1,2-a]chinoxalin-2-carbonsaure
c. 4,5-Dihydro-1-methyl-7-trifluormethyl-4-oxo-imidazolo[1,2-a]chinoxalin-2-carbonsäure
d. 4,5-Dihydro-7,8-dichlor-1-methyl-4-oxo-imidazolo[1,2-a]chinoxalin-2-carbonsäure
e. 4,5-Dihydro-8-chlor-1-methyl-7-trifluormethyl-4-oxo-imidazolo[1,2-a]chinoxalin-2-carbonsäure
f. 4, 5-Dihydro-8-chlor-1-isopropyl-trifluormethyl-4-oxo-imidazolo[1,2-a]chinoxalin-2-carbonsäure
g. 4,5-Dihydro-8-chlor-1-methyl-7-trifluormethyl-4-oxo-imidazolo[1,2-a]chinoxalin-2-carbonsäureethylester
h. 4,5-Dihydro-1-methyl-7-trifluormethyl-4-oxo-imidazolo[1,2-a]chinoxalin-2-carbonsäureethylester
i. 4,5-Dihydro-1-isopropyl-7-trifluormethyl-4-oxo-imidazolo[1,2-a]chinoxalin-2-carbonsäure
j. 4,5-Dihydro-1-methyl-7-trifluormethyl-4-oxo-imidazolo[1,2-a]chinoxalin-2-acrylsäure
k. 4,5-Dihydro-8-chlor-1-ethyl-7-trifluormethyl-4-oxo-imidazolo[1,2-a]chinoxalin-2-carbonsäuremethylester
l. 4,5-Dihydro-8-chlor-1-ethyl-7-trifluormethyl-4-oxo-imidazolo[1,2-a]chinoxalin-2-carbonsäure
m. 4,5-Dihydro-1-methyl-7-trifluormethyl-4-oxo-imidazolo[1,2-a]chinoxalin-2-propionsäure
n. 4,5-Dihydro-8-chlor-7-trifluormethyl-4-oxo-imidazolo[1,2-a]chinoxalin-2-carbonsäure
o. 4,5-Dihydro-1-phenyl-7-trifluormethyl-4-oxo-imidazolo[1,2-a]chinoxalin-2-carbonsäureethylester
p. 4,5-Dihydro-1-phenyl-7-trifluormethyl-4-oxo-imidazolo[1,2-a]chinoxalin-2-carbonsäure
q. 4,5-Dihydro-1-methyl-8-nitro-7-trifluormethyl-4-oxo-imidazolo[1,2-a]chinoxalin-2-carbonsäureethylester
r. 4,5-Dihydro-1-methyl-8-nitro-7-trifluormethyl-4-oxo-imidazolo[1,2-a]chinoxalin-2-carbonsäure
s. 4,5-Dihydro-1-methyl-8-nitro-7-trifluormethyl-4-oxo-imidazolo[1,2-a]chinoxalin-2-propionsäuremethylester
t. 4,5-Dihydro-1-methyl-8-nitro-7-trifluormethyl-4-oxo-imidazolo[1,2-a]chinoxalin-2-propionsäure
u. 4,5-Dihydro-1-methyl-7-nitro-4-oxo-imidazolo[1,2-a]chinoxalin-2-propionsäure
v. 4,5-Dihydro-8-chlor-1-methyl-7-trifluormethyl-4-oxo-imidazolo[1,2-a]chinoxalin-2-propionsäureethylester
w. 4,5-Dihydro-8-chlor-1-methyl-7-trifluormethyl-4-oxo-imidazolo[1,2-a]chinoxalin-2-propionsäure
x. 4,5-Dihydro-1-phenyl-7-trifluormethyl-4-oxo-imidazolo[1,2-a]chinoxalin-2-propionsäure
y. 4,5-Dihydro-8-chlor-1-methyl-7-trifluormethyl-4-oxo-imidazolo[1,2-a]chinoxalin-2-propionsäureethylester

Die Herstellung der Verbindungen der Formel I kann in an sich bekannter Weise nach verschiedenen Methoden durchgeführt werden.

Analog dem in EP 400 583 beschriebenen Verfahren kann man ein Imidazol der Formel II 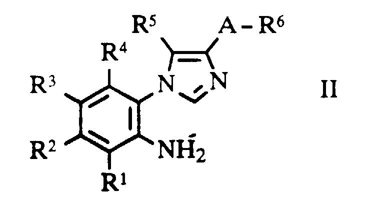 worin R¹ - R⁵ und A die angegebene Bedeutung haben und R⁶ eine Nitril-, Carbonsäureester-, Aldehyd-, oder Alkanoylgruppe bedeutet, mit einem doppelt aktivierten Kohlensäurederivat, wie Phosgen, Diphenylcarbonat oder vorzugsweise Carbonyldiimidazol, in einem inerten, aprotischen Lösungsmittel bei erhöhter Temperatur von 150 bis 200°C umsetzen.

Geeignete Lösungsmittel sind Tetralin, Dekalin, 1,2-Dichlorbenzol oder 1,3-Dimethylethylen- oder -propylenharnstoff.

Hierbei erhält man Verbindungen der Formel I in der R⁶ eine Nitril-, Formyl-, Carbonester- oder Alkanoylgruppe bedeutet.

Bevorzugte Verbindungen sind dabei solche, in denen R¹ - R⁴ und A die oben bereits genannten Bedeutungen besitzen und R⁶ den Rest einer Carbonestergruppe darstellt.

Verbindungen der Formel I, in welcher R⁶ eine Estergruppe bedeutet, können einer sauren oder alkalischen Hydrolyse unterworfen werden, wobei man Verbindungen der Formel I erhält in der R6 eine Carboxygruppe darstellt.

Die Hydrolyse erfolgt vorzugsweise unter alkalischen Bedingungen, beispielsweise in Gegenwart eines Alkalimetallhydroxids oder von Natriumhydrogencarbonat in einem Lösungsmittel, wie Wasser, einem niederen Alkohol, Tetrahydrofuran oder Mischungen derselben. Die so erhaltenen organischen Säuren werden gegebenenfalls in ein physiologisch verträgliches Amin- oder Metallsalz Überführt. Darunter versteht man insbesondere Salze der Alkalimetalle, wie Natrium und Kalium, der Erdalkalimetalle, wie Calcium, sonstiger Metalle, wie Aluminium, sowie Salze von organischen Basen, wie Morpholin, Piperidin, Mono-, Di- und Triethanolamin oder Tris-(hydroxymethyl)aminomethan.

Die Ausgangsverbindungen für das Verfahren Ia können gemäß folgendem Syntheseschema erhalten werden:

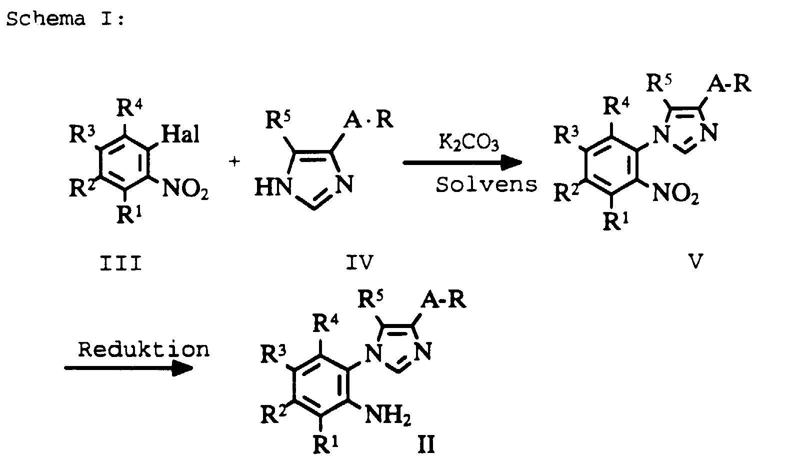

Es ist bekannt, daß sich ortho-halogensubstituierte Nitrobenzole mit am Stickstoffatom N₁ nicht substituierten Imidazolen in geeigneten Lösungsmitteln, wie Dimethylsulfoxid, Dimethylformamid oder Acetonitril bei Temperaturen zwischen 0 und 140°C und unter Basenzusatz, z.B. Kaliumcarbonat, umsetzen lassen.

Es ist weiter bekannt, daß die Substitution des Halogenatoms durch 4- und 4,5-disubstituierte Imidazole so erfolgt, daß der nucleophile Angriff an dem am wenigsten sterisch gehinderten N-Atom des Imidazols erfolgt, so daß in diesem Falle einheitliche Produkte entstehen.

Die Reduktion der Nitroverbindungen kann in an sich bekannter Weise beispielsweise durch katalytische Hydrierung mit Palladium oder Nickelkatalysatoren oder auch mit Zinn-II-chlorid erfolgen.

o-Halogen-nitrobenzole der Formel III sind käuflich erhältlich oder können nach bekannten Methoden hergestellt werden.

Verbindungen der Formel I, worin R⁶ eine Hydroxyalkyl-, Aldehyd-, Oxim- oder Oximethergruppe bedeutet, werden durch die nachfolgend beschrieben Operationen hergestellt. Diese bestehen darin, daß man Verbindungen der Formel I, in denen R⁶ eine Carbonsäureestergruppe bedeutet, reduziert, so daß Verbindungen der Formel I erhalten werden, in welcher R⁶ eine Hydroxyalkylgruppe darstellt.

Diese Reduktion kann mit Hilfe eines komplexen Metallhydrids, wie beispielsweise Lithiumborhydrid, in einem geeigneten Lösungsmittel, wie Ether oder Tetrahydrofuran durchgeführt werden. Die Reduktion wird vorzugsweise bei erhöhter Temperatur, besonders beim Siedepunkt des Lösungsmittels durchgeführt.

Falls erwünscht, können diese Hydroxyalkylverbindungen durch Oxidation mit Hilfe eines Oxidationsmittels wie Chromoxid oder Magandioxid, in Aldehyde der Formel I (R⁶ = Formylgruppe) überführt werden.

Oxime und Oximether können aus den Aldehyden der Formel I durch Umsetzung mit Hydroxylamin und Hydroxylamin-O-alkylethern erhalten werden.

Verbindungen der Formel I in welcher R⁶ eine gegebenenfalls substituierte Carbamoyl- oder eine Carbonylaminotetrazolgruppe bedeutet, werden dadurch erhalten, daß man eine Säure oder deren aktivierte Form der Formel I, wobei R⁶ einen Carbonsäurerest darstellt, mit Ammoniak, Aminen oder 5-Amino-tetrazol umsetzt.

Als Amine eignen sich besonders C₁-C₆-Alkylamine und durch eine Nitro- oder CF₃-Gruppe oder 1-2 C₁₋₄-Alkylgruppen, C₁₋₄-Alkoxygruppen oder Halogenatome am Phenylring substituiertes Anilin, Benzylamin und Phenethylamin.

Für diese Umsetzung wird entweder das Säurechlorid eingesetzt oder die Umsetzung wird in Gegenwart von Carbonyldiimidazol bewirkt. Bevorzugt wird hierbei in Dimethylformamid gearbeitet.

Verbindungen der Formel I mit einem Carbonylaminotetrazolrest für R⁶ (R⁶ = CO-NH-CHN₄) können nach bekannten Methoden durch Kondensation der zugrundeliegenden Carbonsäure mit 5-Aminotetrazol der Formel IV 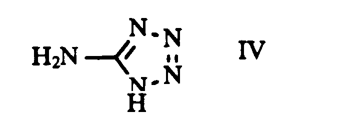 erhalten werden. Die Reaktion wird in der Regel in einem inerten Lösungsmittel, wie beispielsweise Methylenchlorid, Dioxan, Tetrahydrofuran oder Dimethylformamid, bevorzugt in Anwesenheit eines aus der Peptidchemie bekannten Kondensationsreagenzes, wie N,N'-Carbonyldiimidazol oder N,N'-Dicyclohexylcarbodiimid, bei Temperaturen von 20°C bis 120°C durchgeführt.

Falls in den Ausgangsverbindungen die Substituenten R¹ und R² noch nicht vorhanden sind, können diese auch nachträglich eingeführt werden. Das kann durch eine elektrophile aromatische Substitution einer erhaltenen Verbindung der Formel I, in der R¹ und/oder R² Wasserstoffatome bedeuten, nach an sich bekannten Methoden geschehen, wie sie beispielsweise in Houben-Weyl Bd. X/1, S. 471 ff, Bd. IX,S. 572 ff, und Bd. V/3, S. 873, beschrieben sind.

Ein Verfahren zur Herstellung von Nitroverbindungen der Formel I worin R¹, R⁴ - R⁶ und A die oben genannte Bedeutung haben und R² oder R₃ Wasserstoffatome, Nitro- oder Alkylgruppen bedeuten, wobei mindestens einer der Reste R₂ oder R₃ eine Nitrogruppe sein muß, ist dadurch gekennzeichnet, daß man eine Verbindung der Formel I, in welcher R¹, R⁴ - R⁶ und A die oben genannte Bedeutung haben und R² oder R³ Wasserstoffatome oder Alkylgruppen bedeuten, wobei mindestens einer der Reste R² oder R³ ein Wasserstoffatom sein muß, mit Salpetersäure oder Schwefelsäure-Kaliumnitrat bei tiefer Temperatur nitriert. Als vorteilhaft hat sich dabei das Arbeiten mit konzentrierter Salpetersäure bei 0°C ergeben.

Die Synthese von Verbindungen der Formel I, worin R¹ - R⁵ und A die oben genannte Bedeutung haben und R6 einen Tetrazolylrest bedeutet, erfolgt nach an sich bekannten Methoden, wie sie beispielsweise in Synth. 1973, 80 beschrieben sind, durch Umsetzung der entsprechenden Nitrile mit Stickstoffwasserstoffsäure oder einem ihrer Salze, beispielsweise mit Alkali- oder Erdalkaliaziden, gegebenenfalls in Anwesenheit von Lewissäuren wie Aluminium- und Zinnchlorid oder von ammoniumchlorid. Bevorzugt ist die Kombination von Natriumazid mit Ammoniumchlorid. Im allgemeinen wird die Reaktion in Anwesenheit eines inerten Lösungsmittels wie Benzol, Tetrahydrofuran oder Dimethylformamid bei Temperaturen zwischen Raumtemperatur und 150°C durchgeführt. Die Tetrazolylverbindungen sind stark sauer und können in üblicher Weise in ihre Salze mit physiologisch verträglichen Amin- oder Metallkationen überführt werden.

Die erfindungsgmäßen Verbindungen I eignen sich als Arzneimittelwirkstoffe für die Humanmedizin und können zur Herstellung von Arzneimitteln zur Behandlung neurodegenerativer Erkrankungen und neurotoxischer Störungen des zentralen Nervensystems sowie zur Herstellung von Spasmolytika, Antiepileptika, Anxiolytika und Antidepressiva verwendet werden.

Die pharmakologische Wirksamkeit der erfindungsgemäßen Verbindungen I wurde an isoliertem Membranmaterial von Rattengroßhirnen untersucht. Hierzu wurde das Membranmaterial in Gegenwart der erfindungsgemäßen Verbindungen mit den radioaktiv markierten Substanzen ³H-2-Amino-3-hydroxy-5-methyl-4-isoxazol-propionsaure (3H-AMPA) und ³H-2-Amino-3-hydroxy-5-methyl-4-isoxazolpropionsäure (³H-AMPA) und ³H-5,7-Dichlorkynurensaure behandelt, wobei sich diese an spezifische Rezeptoren (AMPA- bzw. NMDA-Rezeptor (N-Methyl-D-aspartat)) binden. Anschließend wurde durch Scintillationszählung die Radioaktivität der behandelten Membranen gemessen. Über die gebundene Radioaktivität ließen sich die Mengen an gebundener ³H-AMPA und ³H-5,7-Dichlorkynurensäure bzw. jeweils die verdrängten Mengen dieser radioaktiv markierten Substanzen bestimmen. Die sich daraus ergebende Dissoziationskonstante K_{I} (I = Inhibitor), welche ein Maß für die Verdrängungswirkung des erfindungsgemäßen Wirkstoffs ist, wurde durch iterative nichtlineare Regressionsanalyse mit dem Statistical Analysis System (SAS) an einem IBM-Rechner, ähnlich dem Programm "Ligand" von P.J. Munson oder D. Rodbard (Analytical Biochm. 107, 220 (1980), Ligand: Versatile Computerized Approach for Charakterization of Ligand Binding Systems) ermittelt.

Folgende In-Vitro-Untersuchungen wurden durchgeführt:
1. Bindung von ³H-2-Amino-3-hydroxy-5-methyl-4-isoxazol-propionsäure (³H-AMPA)
   Für die Präparation des Membranteils wurden frisch entnommene Rattengroßhirne zusammen mit dem ca. 15fachen Volumen einer Pufferlösung A aus 30 mM a,a,a-Tris-(hydroxymethyl)-methylamin-Hydrochlorid (TRIS-HCl) und 0,5 mM Ethylendiamintetraessigsäure (EDTA) - pH 7,4 -mittels eines Ultra-TURRAX-Rührers homogenisiert. Nach Abtrennung der überstehenden Flüssigkeit wurde das im Bodensatz enthaltene proteinhaltige Membranmaterial dreimal durch Suspendieren in der Pufferlösung A und anschließendes, jeweils 20 min Zentrifugieren bei 48 000 g gewaschen. Danach wurde das Membranmaterial in einem 15fachen Volumen der Pufferlösung A suspendiert und 30 min bei 37°C inkubiert. Anschließend wurde das Proteinmaterial zweimal durch Zentrifugieren und Suspendieren gewaschen und bis zur Verwendung bei -70°C eingefroren.
   Für den Bindungstest wurde das bei 37°C aufgetaute Proteinmaterial zweimal durch Zentrifugieren bei 48 000 g (20 min) und anschließendes Suspendieren in einer Pufferlösung B aus 50 mM TRIS-HCl, 0,1 M Kaliumthiocyanat und 2,5 mM Calciumchlorid- pH 7,1 - gewaschen. Anschließend wurden 0,25 mg Membranmaterial, 0,1 µCi ³H-AMPA (60 Ci/mmol) sowie Verbindung I in 1 ml Pufferlösung B gelöst und 60 min auf Eis inkubiert. Die inkubierte Lösung wurde über einen CF/B-Filter (Firma Whathman), der zuvor mindestens 2 h mit einer 0,5 %igen wäßrigen Lösung von Polyethylenimin behandelt worden war, filtriert. Anschließend wurde das Filtrat mit 5 ml kalter Pufferlösung B gewaschen, um gebundene und freie ³H-AMPA voneinander zu trennen. Nach Messung der Radioaktivität der gebundenen ³H-AMPA im Membranmaterial durch Scintillationszählung wurde durch Auswertung der Verdrängungskurven mittels Regressionsanalyse der K_{I}-Wert bestimmt.
2. Bindung von ³H-5,7-Dichlorkynurensäure
   Für die Präparation des Membranmaterials wurden frisch entnommene Rattengroßhirne zusammen mit dem 10fachen Volumen einer Pufferlösung A' aus 50 mM TRIS-HCl und 10 mM EDTA - pH 7,4 - homogenisiert. Die Suspension wurde 20 min bei 48 000 g zentrifugiert. Nach Abtrennung der überstehenden Flüssigkeit wurde das im Bodensatz enthaltene Membranmaterial zweimal durch Suspendieren in der Pufferlösung A' und anschließendes jeweils 20minütiges Zentrifugieren und Suspendieren gewaschen. Nach erneutem Suspendieren der Membranen in der PufferLösung A' und Einfrieren in flüssigem Stickstoff wurde die Suspension wieder bei 37°C aufgetaut und nach einem weiteren Waschvorgang 15 min bei 37°C inkubiert.
   Anschließend wurde das Proteinmaterial viermal durch Zentrifugieren und Suspendieren gewaschen und bis zur Verwendung bei -70°C eingefroren.
   Für den Bindungstest wurde das bei 37°C aufgetaute Proteinmaterial zweimal durch Zentrifugieren bei 48 000 g (20 min) und anschließendes Suspendieren in einer Pufferlösung B' aus 50 mM TRIS-HCl - pH 7,4 - gewaschen. Anschließend wurden 0,15 mg Membranmaterial, 0,3 µCi ³H-5,7-Dichlorkynurensäure (16 Ci/mmol) sowie Verbindung I in 1 ml Pufferlösung B' gelöst und 30 min auf Eis inkubiert. Die inkubierte Lösung wurde 2 min bei 150 000 g zentrifugiert. Nach Abtrennung der überstehenden Flüssigkeit wurden die Bodensatze zweimal mit je 1,5 ml kalter Pufferlösung B' suspendiert. Nach Messung der Radioaktivität der an die Membranen gebundenen ³H-5,7-Dichlorkynurensäure im Bodensatz ergab sich der K_{I}-Wert durch Auswertung der Verdrängungskurven mittels der Regressionsanalyse.

Die Herstellung der Arzneimittelzubereitungen erfolgt in üblicher Weise, z.B. durch Vermischen des Wirkstoffes mit den anderen-üblichen Trägerstoffen und Verdünnungsmitteln.

Die Arzneimittelzubereitungen können in verschiedenen Applikationsweisen verabreicht werden, wie peroral, parenteral, subkutan, intraperitonal und topisch. So sind Zubereitungsformen wie Tabletten, Emulsionen, Infusions- und Injektionslösungen, Pasten, Salben, Gele, Cremes, Lotionen, Puder und Sprays möglich.

Die erfindungsgemäßen Arzneimittelzubereitungen enthalten neben den üblichen Arzneimittelhilfsstoffen eine therapeutisch wirksame Menge der Verbindung I. Für die lokale äußere Anwendung, z.B. in Puder und Salben, können die Wirkstoffe in den üblichen Konzentrationen enthalten sein. In der Regel sind die Wirkstoffe in einer Menge von 0,001 bis 5 Gew.-%, vorzugsweise 0,02 bis 0,5 Gew.-% enthalten.

Bei der inneren Anwendung werden die Präparationen in Einzeldosen verabreicht. In einer Einzeldosis werden pro kg Körpergewicht 0,1 bis 50 mg, vorzugsweise 0,1 bis 10 mg Wirkstoff gegeben. Die Zubereitungen können täglich in einer oder mehreren Dosierungen je nach Art und Schwere der Erkrankungen verabreicht werden. Die Tagesdosis liegt in der Regel bei 0,1 bis 20 mg pro kg Körpergewicht bei oraler Gabe bzw. 0,01 bis 10 mg pro kg Körpergewicht bei parenteraler Gabe.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes hydriertes Ricinusöl, Polyacrylsäure, Polyethylenglykol, Polyethylenglykolstearat, ethoxylierte Fettalkohole, Paraffinöl, Vaseline und Wollfett verwendet werden. Für die innere Anwendung eignen sich z.B. Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Bleichmittel enthalten sein.

Die neben dem Wirkstoff in der Zubereitung enthaltenen Stoffe sowie die bei der Herstellung der pharmazeutischen Zubereitung verwendeten Stoffe müssen toxikologisch unbedenklich und mit dem jeweiligen Wirkstoff verträglich sein.

Die folgenden Beispiele erläutern die Erfindung naher.

### Beschreibung der Versuche

### A Herstellung der Ausgangsprodukte

### a. 1-(2-Nitrophenyl)-imidazole

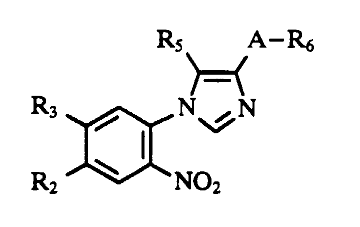

### 1) 1-(2-Nitro-4-trifluormethylphenyl)-5-carbethoxy-4-methyl-imidazol

Eine Mischung von 10,45 g (0,05 Mol) 2-Fluor-4-trifluormethylnitrobenzol, 7,7 g (0,05 Mol) 4(5)-Carbethoxy-5(4)-methylimidazol und 13,8 g Kaliumcarbonat wurden in 100 ml Acetonitril 4 h unter Rühren zum Sieden erhitzt.

Die abgekühlte Reaktionsmischung wurde mit 1000 ml Wasser versetzt, mit 250 ml Methylenchlorid extrahiert und die Methylenchloridphase mit Magnesiumsulfat getrocknet. Die getrocknete Lösung wurde eingedampft und der Rückstand durch Anreiben mit Ether zur Kristallisation gebracht. Die Substanz ist für die nachfolgenden Umsetzungen genügend rein.
Ausbeute: 11,4 g (66 % d. Th.)
Fp.: 142 - 144°C

Durch Variation des Nitrobenzolderivates, des Imidazols, des Lösungsmittels, der Temperatur und der Reaktionszeit wurden die nachfolgenden Verbindungen erhalten.

| Nr. | R₂ | R₃ | R₅ | A | R₆ | Fp. °C |
|---|---|---|---|---|---|---|
| 2) | H | H | CH₃ | - | COOEt | 94-96 |
| 3) | H | CH₃ | CH₃ | - | COOEt | 110 |
| 4) | H | F | CH₃ | - | COOEt | 122-124 |
| 5) | H | H | H | CH₂ | COOEt | Öl |
| 6) | Cl | H | CH₃ | - | COOEt | 112-115 |
| 7) | CF₃ | Cl | CH₃ | - | COOEt | 118-119 |
| 8) | Cl | H | H | - | COCH₃ | 151 |
| 9) | CF₃ | H | CH₃ | - | COOEt | 142-144 |
| 10) | H | i-BuO | CH₃ | - | COOEt | 65 |
| 11) | Cl | Cl | CH₃ | - | COOEt | 152 |
| 12) | H | H | C₂H₅ | - | COOMe | 110-113 |
| 13) | COOEt | H | CH₃ | - | COOEt | 119 |
| 14) | CF₃ | H | i-C₃H₇ | - | COOEt | Öl |
| 15) | CF₃ | Cl | C₂H₅ | - | COOMe | 142-144 |
| 16) | CH₃ | CH₃ | CH₃ | - | COOEt | 128-132 |
| 17) | CF₃ | H | CH₃ | -CH=CH- | COOMe | 168-172 |
| 18) | Cl | Cl | H | - | COOMe | 160-163 |
| 19) | CF₃ | H | H | - | COOMe | 146-150 |
| 20) | NO₂ | H | CH₃ | - | COOEt | 128-131 |
| 21) | CF₃ | Cl | H | - | COOMe | 99-103 |
| 22) | F | Br | CH₃ | - | COOEt | 140-145 |
| 23) | Br | CH₃ | CH₃ | - | COOEt | 133-134 |
| 24) | Br | H | CH₃ | - | COOEt | 125-130 |
| 25) | H | Cl | CH₃ | -CH=CH- | COOEt | 128-129 |
| 26) | CF₃ | Cl | H | -CH₂ - | COOEt | 122-123 |
| 27) | CF₃ | H | C₆H₅ | -CH=CH- | COOEt | 164-165 |
| 28) | CF₃ | Cl | CH₃ | -CH=CH- | COOEt | 208-209 |
| 29) | CF₃ | H | CH₃ | -CH=CH- | COOMe | 168-173 |
| 30) | (CH₃)₂NSO₂H | | CH₃ | - | COOEt | 248-250 |
| 31) | CF₃ | H | C₆H₅ | - | COOEt | 138-141 |
| 32) | CF₃O | H | CH₃ | - | COOEt | 98-101 |
| 33) | CH₃CONH | H | CH₃ | - | COOEt | 129-134 |
| 34) | i-Bu | H | CH₃ | - | COOEt | Öl |
| 35) | CF₃ | Cl | CH₃ | -CH₂CH₂ - | COOEt | 185-190 |
| 36) | Cl | (6-Cl) | CH₃ | - | COOEt | 143-144 |
| 37) | F | Br | CH₃ | - | COOEt | 133-134 |

### b. 1-(2-Aminophenyl)-imidazole

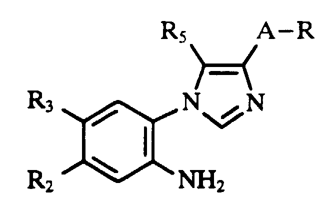

### 1) 1- (2-Amino-4-trifluormethylphenyl)-5-carbethoxy-4-methyl-imidazol

11,6 g (0,034 Mol) 1-(2-Nitro-4-trifluormethylphenyl)-5-carbethoxy-4-methylimidazol (vgl. Aa1) wurden mit 2 g Palladium-Kohle-Katalysator (10 % Pd) in 100 ml Ethanol bei Raumtemperatur unter Atmosphärendruck hydriert. Nach Beendigung der Wasserstoffaufnahme wurde die vom Katalysator befreite Lösung im Vakuum eingedampft und der verbleibende Rückstand mit etwas Ether zur Kristallisation gebracht.
Ausbeute: 9,8 g (93 % d. Th.)
Fp.: 189 - 190°C

In analoger Weise wurden die nachstehend aufgeführten Verbindungen erhalten, wobei für chlorhaltige Verbindungen Raney-Nickel als Katalysator verwendet wurde.

| Nr. | R₂ | R₃ | R₅ | A | R₆ | Fp. °C |
|---|---|---|---|---|---|---|
| 2) | H | H | CH₃ | - | COOEt | 139 |
| 3) | H | CH₃ | CH₃ | - | COOEt | 123 |
| 4) | H | F | CH₃ | - | COOEt | 188 |
| 5) | H | H | H | CH₂ | COOEt | Öl |
| 6) | Cl | H | CH₃ | - | COOEt | 165 |
| 7) | CF₃ | Cl | CH₃ | - | COOEt | 202-205 |
| 8) | Cl | H | H | - | COCH₃ | 210 |
| 9) | H | i-BuO | CH₃ | - | COOEt | Öl |
| 10) | Cl | Cl | CH₃ | - | COOEt | 224 |
| 11) | H | H | C₂H₅ | - | COOEt | 141-143 |
| 12) | COOEt | H | CH₃ | - | COOCH₃ | 158-160 |
| 13) | CF₃ | H | i-C₃H₇ | - | COOEt | 98-103 |
| 14) | CF₃ | Cl | C₂H₅ | - | COOEt | 196-198 |
| 15) | CH₃ | CH₃ | CH₃ | - | COOEt | 154-158 |
| 16) | Cl | Cl | H | - | COOMe | 197-200 |
| 17) | CF₃ | H | H | - | COOMe | 209-212 |
| 18) | NO₂ | H | CH₃ | - | COOEt | 114-117 |
| 19) | CF₃ | Cl | H | - | COOMe | 237-241 |
| 20) | CF₃ | H | CH₃ | -CH₂-CH₂- | COOMe | 99-103 |
| 21) | F | Br | CH₃ | - | COOEt | 172-174 |
| 22) | Br | CH₃ | CH₃ | - | COOEt | 204-206 |
| 23) | Br | H | CH₃ | - | COOEt | 190-195 |
| 24) | t-Bu | H | CH₃ | - | COOEt | 143-144 |
| 25) | Cl | (6-Cl) | CH₃ | - | COOEt | 189-190 |
| 26) | i-Bu | H | CH₃ | - | COOEt | 143-145 |
| 27) | CH₃CONH | H | CH₃ | - | COOEt | 100-103 |
| 28) | CF₃O | H | CH₃ | - | COOEt | 157-160 |
| 29) | CF₃ | H | C₆H₅ | - | COOEt | 273-275 |
| 30) | (CH₃)₂NSO₂H | | CH₃ | - | COOEt | 248-250 |
| 31) | NO₂ | H | CH₃ | -CH₂-CH₂- | COOEt | 163-164 |
| 32) | CF₃ | Cl | CH₃ | -CH₂-CH₂- | COOEt | 128-129 |
| 33) | CF₃ | H | C₆H₅ | -CH₂-CH₂- | COOEt | 114-116 |
| 34) | CF3 | Cl | H | -CH2- | COOEt | 151-152 |
| 35) | H | Cl | CH₃ | -CH2-CH2- | COOEt | 112-113 |

### B. Herstellung der Endprodukte

Umsetzung von 1-(2-Aminophenyl)-imidazolen mit Carbonyldiimidazol:

### Beispiele 1

4,5-Dihydro-1-methyl-4-oxo-imidazolo[1,2-a]chinoxalin-2-carbonsäureethylester

3,5 g (0,143 Mol) 1-(2-Aminophenyl)-4-carbethoxy-5-methylimidazol (0,154 Mol) Carbonyldiimidazol wurden unter Rühren in 50 ml 1,2-Dichlorbenzol 1,5 h zum Sieden erhitzt.

Nach dem Abkühlen wurde der Festkörper abgesaugt, mit Aceton gewaschen und das Produkt aus DMF umkristallisiert.
Ausbeute: 3,0 g (77 % d. Th.)
C₁₄H₁₃N₃O₃ MG 271 Fp.: > 300°C

Analog wurden erhalten:
2. 4,5-Dihydro-1,8-dimethyl-4-oxo-imidazolo[1,2-a]chinoxalin-2-carbonsäureethylester
   C₁₅H₁₅N₃O₃ MG 285 Fp. 255-257°C
3. 4,5-Dihydro-8-fluor-1-methyl-4-oxo-imidazolo[1,2-a]chinoxalin-2-carbonsäureethylester
   C₁₄H₁₂FN₃O₃ MG 289 Fp. 255-257°C
4. 4,5-Dihydro-1-methyl-7-trifluormethyl-4-oxo-imidazolo[1,2-a]chinoxalin-2-carbonsäureethylester
   C₁₅H₁₂F₃N₃O₃ MG 339 Fp. 270-271°C
5. 4,5-Dihydro-8-isobutoxy-1-methyl-4-oxo-imidazolo[1,2-a]chinoxalin-2-carbonsäureethylester
   C₁₈H₂₁N₃O₄ MG 343 Fp. 190-195°C
6. 4,5-Dihydro-7-chlor-1-methyl-4-oxo-imidazolo[1,2-a]chinoxalin-2-carbonsaureethylester
   C₁₄H₁₂ClN₃O₃ MG 305 Fp. 277-279°C
7. 4,5-Dihydro-4-oxo-imidazolo[1,2-a]chinoxalin-2-essigsäureethylester
   C₁₄H₁₃N₃O₃ MG 271 Fp. 265-271°C
8. 4,5-Dihydro-1-ethyl-4-oxo-imidazolo[1,2-a]chinoxalin-2-carbonsäuremethylester
   C₁₄H₁₃N₃O₃ MG 271 Fp. 264-266°C
9. 4,5-Dihydro-2-acetyl-7-chlor-4-oxo-imidazolo[1,2-a]chinoxalin
   C₁₂H₈ClN₃O₂ MG 261 Fp. > 320°C
10. 4,5-Dihydro-1-methyl-4-oxo-imidazolo[1,2-a]chinoxalin-2,7-dicarbonsäure-diethylester
   C₁₇H₁₇N₃O₅ MG 343 Fp. 295-300°C
11. 4,5-Dihydro-8-chlor-1-methyl-7-trifluormethyl-4-oxo-imidazolo[1,2-a]chinoxalin-2-carbonsäureethylester
   C₁₅H₁₁ClF₃N₃O₃ MG 373 Fp. 310-315°C
12. 4,5-Dihydro-1-isopropyl-7-trifluormethyl-4-oxo-imidazolo[1,2-a]chinoxalin-2-carbonsäureethylester
   C₁₇H₁₆F₃N₃O₃ MG 367 Fp. 215-220°C
13. 4,5-Dihydro-1-methyl-4-oxo-imidazolo[1,2-a]chinoxalin-2-acrylsäuremethylester
   C₁₅H₁₃N₃O₃ MG 283 Fp. > 300°C
14. 4,5-Dihydro-7,8-dichlor-1-methyl-4-oxo-imidazolo[1,2-a]chinoxalin-2-carbonsäureethylester
   C₁₄H₁₁Cl₂N₃O₃ MG 340 Fp. 285°C (Z)
15. 4,5-Dihydro-1-methyl-7-trifluormethyl-4-oxo-imidazolo[1,2-a]chinoxalin-2-acrylsäureethylester
   C₁₇H₁₄F₃N₃O₃ MG 365 Fp. > 280°C
16. 4,5-Dihydro-8-chlor-1-ethyl-7-trifluormethyl-4-oxo-imidazolo[1,2-a]chinoxalin-2-carbonsaureethylester
   C₁₅H₁₁ClF₃N₃O₃ MG 373 Fp. 304-308°C
17. 4,5-Dihydro-1-methyl-7-nitro-4-oxo-imidazolo[1,2-a]chinoxalin-2-carbonsäureethylester
   C₁₄H₁₂N₄O₅ MG 316 Fp. > 300°C
18. 4,5-Dihydro-1-methyl-7-trifluormethyl-4-oxo-imidazolo[1,2-a]chinoxalin-2-propionsäuremethylester
   C₁₆H₁₄F₃N₃O₃ MG 353 Fp. 203-206°C
19. 4,5-Dihydro-8-chlor-7-trifluormethyl-4-oxo-imidazolo[1,2-a]chinoxalin-2-carbonsäuremethylester
   C₁₃H₇ClF₃N₃O₃ MG 345 Fp. 270-280°C
20. 4,5-Dihydro-7-trifluormethyl-4-oxo-imidazolo[1,2-a]chinoxalin-2-carbonsäuremethylester
   C₁₃H₈F₃N₃O₃ MG 311 Fp. 281-283°C
21. 4,5-Dihydro-8-brom-7-fluor-1-methyl-4-oxo-imidazolo[1,2-a]chinoxalin-2-carbonsäureethylester
   C₁₄H₁₁BrFN₃O₃ MG 368 Fp. 298-300°C
22. 4,5-Dihydro-7-isobutyl-1-methyl-4-oxo-imidazolo[1,2-a]chinoxalin-2-carbonsäureethylester
   C₁₈H₂₁N₃O₃ MG327 Fp. 270-273°C
23. 4,5-Dihydro-7-acetamido-1-methyl-4-oxo-imidazolo[1,2-a]chinoxalin-2-carbonsäureethylester
   C₁₆H₁₆N₄O₄ MG 328 Fp. > 300°C
24. 4,5-Dihydro-7-brom-1-methyl-4-oxo-imidazolo[1,2-a]chinoxalin-2-carbonsäureethylester
   C₁₄H₁₂BrN₃O₃ MG 350 Fp. > 300°C
25. 4,5-Dihydro-1-methyl-7-trifluormethoxy-4-oxo-imidazolo[1,2-a]chinoxalin-2-carbonsäureethylester
   C₁₅H₁₂F₃N₃O₄ MG 355 Fp. > 300°C
26. 4,5-Dihydro-1-phenyl-7-trifluormethyl-4-oxo-imidazolo[1,2-a]chinoxalin-2-carbonsaureethylester
   C₂₀H₁₄F₃N₃O₃ MG 401 Fp. 292-295°C
27. 4,5-Dihydro-8-chlor-1-methyl-7-trifluormethyl-4-oxo-imidazolo[1,2-a]chinoxalin-2-propionsaureethylester
   C₁₇H₁₅ClF₃N₃O₃ MG 402 Fp. 233-235°C
28. 4,5-Dihydro-8-chlor-1-methyl-4-oxo-imidazolo[1,2-a]chinoxalin-2-propionsäureethylester
   C₁₆H₁₆ClN₃O₃ MG 334 Fp. 291-293°C
29. 4,5-Dihydro-8-chlor-7-trifluormethyl-4-oxo-imidazolo[1,2-a]chinoxalin-2-essigsäureethylester
   C₁₅H₁₁ClF₃N₃O₃ MG 373 Fp. > 300°C
30. 4,5-Dihydro-7-dimethylaminosulfonyl-1-methyl-4-oxo-imidazolo[1,2-a]chinoxalin-2-carbonsäureethylester
   C₁₆H₁₈N₄O₅S MG 378 Fp. >300°C
31. 4,5-Dihydro-1-methyl-7-nitro-4-oxo-imidazolo[1,2-a]chinoxalin-2-propionsäureethylester
   C₁₆H₁₆N₄O₅ MG 344 Fp. 250-255°C
32. 4,5-Dihydro-1-phenyl-7-trifluormethyl-4-oxo-imidazolo[1,2-a]chinoxalin-2-propionsäureethylester
   C₂₂H₁₈F₃N₃O₃ MG 429 Fp. 192-193°C
33. 4,5-Dihydro-1,7,8-trimethyl-4-oxo-imidazolo[1,2-a]chinoxalin-2-carbonsäureethylester
   C₁₆H₁₇N₃O₃ MG 299 Fp. > 300°C
34. 4,5-Dihydro-7-t-butyl-1-methyl-4-oxo-imidazolo[1,2-a]chinoxalin-2-carbonsäureethylester
   C₁₈H₂₁N₃O₃ MG 327 Fp. 184-185°C
35. 4,5-Dihydro-7,9-dichlor-1-methyl-4-oxo-imidazolo[1,2-a]chinoxalin-2-carbonsäureethylester
   C₁₄H₁₁Cl₂N₃O₃ MG 340 Fp. 261-267°C

Hydrolyse von Imidazolochinoxalin-carbonestern:

### Beispiel 36

### 4,5-Dihydro-1-methyl-4-oxo-imidazolo[1,2-a]chinoxalin-2-carbonsäure

29 g (0,107 Mol) des gemäß Beispiel 1 erhaltenen Esters wurden mit 500 ml 2-N-Natronlauge und 300 ml Ethanol 1,5 h bei 80°C gerührt. Nach dem Abkühlen wurde mit konzentrierter Salzsäure angesäuert und das Produkt abgesaugt. Es wurde aus DMF umkristallisiert.
Ausbeute: 23 g (88 % d. Th.)
C₁₂H₉N₃O₃ MG 243 Fp.: > 300°C

Auf analoge Weise wurden erhalten:
37. 4,5-Dihydro-1,8-dimethyl-4-oxo-imidazolo [1,2-a]chinoxalin-2-carbonsäure
   C₁₃H₁₁N₃O₃ MG 257 Fp. > 300°C
38. 4,5-Dihydro-8-fluor-1-methyl-4-oxo-imidazolo[1,2-a]chinoxalin-2-carbonsäure
   C₁₂H₈FN₃O₃ MG 261 Fp. > 300°C
39. 4,5-Dihydro-1-methyl-7-trifluormethyl-4-oxo-imidazolo[1,2-a]chinoxalin-2-carbonsäure
   C₁₃H₈F₃N₃O MG 311 Fp. > 300°C
40. 4,5-Dihydro-8-isobutoxy-1-methyl-4-oxo-imidazolo[1,2-a]chinoxalin-2-carbonsäure
   C₁₆H₁₇N₃O₄ MG 315 Fp. 270-275°C
41. 4,5-Dihydro-1-ethyl-4-oxo-imidazolo[1,2-a]chinoxalin-2-carbonsäure
   C₁₃H₁₁N₃O₃ MG 257 Fp. 335-340°C
42. 4,5-Dihydro-7-chlor-1-methyl-4-oxo-imidazolo[1,2-a]chinoxalin-2-carbonsäure
   C₁₂H₈ClN₃O₃ MG 277 Fp. > 320°C
43. 4,5-Dihydro-4-oxo-imidazolo[1,2-a]chinoxalin-2-essigsäure
   C₁₂H₉N₃O₃ MG 243 Fp. 320-325°C
44. 4,5-Dihydro-8-chlor-1-methyl-7-trifluormethyl-4-oxo-imidazolo[1,2-a]chinoxalin-2-carbonsäure
   C₁₃H₇ClF₃N₃O₃ MG 345 Fp. > 300°C
45. 4,5-Dihydro-1-isopropyl-7-trifluormethyl-4-oxo-imidazolo[1,2-a]chinoxalin-2-carbonsäure
   C₁₅H₁₂F₃N₃O₃ MG 339 Fp. 245-250°C
46. 4,5-Dihydro-1-methyl-4-oxo-imidazolo[1,2-a]chinoxalin-2-acrylsäure
   C₁₄H₁₁N₃O₃ MG 269 Fp. > 300°C
47. 4,5-Dihydro-7,8-dichlor-1-methyl-4-oxo-imidazolo[1,2-a]chinoxalin-2-carbonsäure
   C₁₂H₇C₁₂N₃O₃ MG 312 Fp. > 300°C
48. 4,5-Dihydro-1-methyl-7-trifluormethyl-4-oxo-imidazolo[1,2-a]chinoxalin-2-acrylsäure
   C₁₅H₁₀F₃N₃O₃ MG 337 Fp. > 320°C
49. 4,5-Dihydro-8-chlor-1-ethyl-7-trifluormethyl-4-oxo-imidazolo[1,2-a]chinoxalin-2-carbonsaure
   C₁₄H₉ClF₃N₃O₃ MG 359 Fp. > 300°C
50. 4,5-Dihydro-1-methyl-7-nitro-4-oxo-imidazolo[1,2-a]chinoxalin-2-carbonsäure
   C₁₂H₈N₄O₅ MG 288 Fp. > 300°C
51. 4,5-Dihydro-7-trifluormethyl-4-oxo-imidazolo[1,2-a]chinoxalin-2-carbonsäure
   C₁₂H₆F₃N₃O₃ MG 297 Fp. > 300°C
52. 4,5-Dihydro-1-methyl-7-trifluormethyl-4-oxo-imidazolo[1,2-a]chinoxalin-2-propionsäure
   C₁₅H₁₂F₃N₃O₃ MG 339 Fp. > 300°C
53. 4,5-Dihydro-1,7,8-trimethyl-4-oxo-imidazolo[1,2-a]chinoxalin-2-carbonsäure
   C₁₄H₁₃N₃O₃ MG 271 Fp. > 300°C
54. 4,5-Dihydro-7-brom-1-methyl-4-oxo-imidazolo[1,2-a]chinoxalin-2-carbonsäure
   C₁₂H₈BrN₃O₃ MG 322 Fp. > 300°C
55. 4,5-Dihydro-8-brom-7-fluor-1-methyl-4-oxo-imidazolo[1,2-a]chinoxalin-2-carbonsäure
   C₁₂H₇BrFN₃O₃ MG 340 Fp. > 300°C
56. 4,5-Dihydro-8-chlor-7-trifluormethyl-4-oxo-imidazolo[1,2-a]chinoxalin-2-carbonsäure
   C₁₂H₅ClF₃N₃O₃ MG 331 Fp. > 300°C
57. 4,5-Dihydro-7-t-butyl-1-methyl-4-oxo-imidazolo[1,2-a]chinoxalin-2-carbonsäure
   C₁₆H₁₇N₃O₃ MG 299 Fp. > 300°C
58. 4,5-Dihydro-7,9-dichlor-1-methyl-4-oxo-imidazolo[1,2-a]chinoxalin-2-carbonsäure
   C₁₂H₇Cl₂N₃O₃ MG 312 Fp. > 300°C
59. 4,5-Dihydro-7-isobutyl-1-methyl-4-oxo-imidazolo[1,2-a]chinoxalin-2-carbonsäure
   C₁₆H₁₇N₃O₃ MG 299 Fp. 256-259°C
60. 4,5-Dihydro-7-acetamido-1-methyl-4-oxo-imidazolo[1,2-a]chinoxalin-2-carbonsäure
   C₁₄H₁₂N₄O₄ MG 300 Fp. > 350°C
61. 4,5-Dihydro-1-methyl-7-trifluormethoxy-4-oxo-imidazolo[1,2-a]chinoxalin-2-carbonsäure
   C₁₃H₈F₃N₃O₄ MG 327 Fp. > 300°C
62. 4,5-Dihydro-1-phenyl-7-trifluormethyl-4-oxo-imidazolo[1,2-a]chinoxalin-2-carbonsäure
   C₁₈H₁₀F₃N₃O₃ MG 373 Fp. > 300°C
63. 4,5-Dihydro-1-methyl-8-nitro-7-trifluormethyl-4-oxo-imidazolo[1,2-a]chinoxalin-2-carbonsäure
   C₁₃H₇F₃N₄O₅ MG 356 Fp. > 300°C
64. 4,5-Dihydro-7-(dimethylaminosulfonyl)-1-methyl-4-oxo-imidazolo[1,2-a]chinoxalin-2-carbonsäure
   C₁₄H₁₄N₄O₅S MG 350 Fp. > 300°C
65. 4,5-Dihydro-1-methyl-8-nitro-7-trifluormethyl-4-oxo-imidazolo[1,2-a]chinoxalin-2-propionsäure
   C₁₅H₁₁F₃N₄O₅ MG 384 Fp. > 300°C
66. 4,5-Dihydro-1-methyl-8-nitro-4-oxo-imidazolo[1,2-a]chinoxalin-2-propionsäure
   C₁₄H₁₂N₄O₅ MG 316 Fp. > 300°C
67. 4, 5-Dihydro-8-chor-1-methyl-7-trifluormethyl-4-oxo-imidazolo[1,2-a]chinoxalin-2-propionsäure
   C₁₅H₁₁ClF₃N₃O₃ MG 373 Fp. > 300°C
68. 4,5-Dihydro-1-phenyl-7-trifluormethyl-4-oxo-imidazolo[1,2-a]chinoxalin-2-propionsäure
   C₂₀H₁₄F₃N₃O₃ MG 401 Fp. 275-279°C
69. 4,5-Dihydro-8-chlor-7-trifluormethyl-4-oxo-imidazolo[1,2-a]chinoxalin-2-essigsäure
   C₁₃H₇ClF₃N₃O₃ MG 345 Fp. > 300°C
70. 4,5-Dihydro-8-chlor-1-methyl-7-nitro-4-oxo-imidazolo[1,2-a]chinoxalin-2-propionsäure
   C₁₄H₁₁ClN₄O₅ MG 351 Fp. > 300°C
71. 4,5-Dihydro-8-chlor-1-methyl-4-oxo-imidazolo[1,2-a]chinoxalin-2-propionsäure
   C₁₄H₁₂ClN₃O₃ MG 306 Fp. > 300°C

### Reduktion von Estern

### Beispiel 72

4,5-Dihydro-2-hydroxymethyl-1-methyl-7-trifluormethyl-4-oxo-imidazolo[1,2-a]chinoxalin

37 g (0,11 Mol) des nach Beispiel 4 erhaltenen 4,5-Dihydro-1-methyl-7-trifluormethyl-4-oxo-imidazolo[1,2-a]chinoxalin-2-carbonsäure-ethylesters wurden mit 3,65g Lithiumborhydrid in 500 g trockenem Tetrahydrofuran 48 h bei Raumtemperatur gerührt und anschließend weitere 5 h zum Sieden erhitzt.

Nach Zugabe von 100 ml 2-N-Salzsäure zu der erkalteten Reaktionsmischung (Gasentwicklung!) wurde das Lösungsmittel im Vakuum abdestilliert, 300 ml Wasser zugefügt und das Produkt abgesaugt. Es wurde aus Dimethylformamid umkristallisiert.
Ausbeute: 26 g (78 % d. Th.)
C₁₃H₁₀F₃N₃O₂ MG 297 Fp. > 300°C

In analoger Weise wurden erhalten:
73. 4,5-Dihydro-1,7-dimethyl-2-hydroxymethyl-4-oxo-imidazolo[1,2-a]chinoxalin
   C₁₃H₁₃N₃O₂ MG 243 Fp. > 300°C
74. 4,5-Dihydro-2-hydroxymethyl-1-methyl-4-oxo-imidazolo[1,2-a]chinoxalin
   C₁₂H₁₁N₃O₂ MG 229 Fp. 231-233°C
75. 4,5-Dihydro-7-chlor-2-hydroxymethyl-1-methyl-4-oxo-imidazolo[1,2-a]chinoxalin
   C₁₂H₁₀ClN₃O₂ MG 263 Fp. > 300°C

Oxidation von Hydroxyalkylverbindungen zu Aldehyden:

### Beispiel 76

### 4,5-Dihydro-1-methyl-7-trifluormethyl-4-oxo-imidazolo[1,2-a]chinoxalin-2-carbaldehyd

20,0 g (0,067 Mol) des nach Beispiel 72 erhaltenen 4,5-Dihydro-2-hydroxymethyl-1-methyl-7-trifluormethyl-4-oxo-imidazolo[1,2-a]chinoxalins wurden mit 58 g aktiviertem Mangandioxid (0,67 Mol) in 500 ml Dimethylformamid 2 h bei 100°C gerührt.

Die heiß filtrierte Lösung wurde anschließend im Vakuum eingedampft, der Rückstand mit Ether angerührt und abgesaugt.
Man erhielt: 17 g (85 % d. Th.)
C₁₃H₈F₃N₃O₂ MG 215 Fp. > 300 °C

Analog wurde erhalten:
77. 4,5-Dihydro-1-methyl-4-oxo-imidazolo[1,2-a]chinoxalin-2-carbaldehyd
   C₁₂H₉N₃O₂ MG 227 Fp. > 300°C

### Herstellung von Oximen und Oximethern

### Beispiel 78

### 4,5-Dihydro-2-hydroximinomethyl-1-methyl-7-trifluormethyl-4-oxoimidazolo[1,2-a]chinoxalin

1,0 g (0,0037 Mol) des nach Beispiel 76 erhaltenen 4,5-Dihydro-1-methyl-7-trifluor-methyl-4-oxo-imidazolo[1,2-a]chinoxalin-2-carbaldehyds wurden zusammen mit 0,47g (0,0067 Mol) Hydroxylaminhydrochlorid, 0,6 g Natriumacetat, 10 ml Wasser und 12,5 ml Ethanol 5 h unter Rühren zum Sieden erhitzt. Nach Abkühlen wurde der Niederschlag abgesaugt und mit Ethanol, Wasser und wenig Aceton gewaschen.
Man erhielt 0,5 g (48 % d. Th.)
C₁₃H₉F₃N₄O₂ MG 310 Fp. 320 - 322°C

In analoger Weise wurden hergestellt:

79. 4,5-Dihydro-2-hydroximinomethyl-1-methyl-4-oxo-imidazolo[1,2-a]chinoxalin
C₁₂H₁₀N₄O₂ MG 242 Fp. 315 - 320°C In prinzipiell ähnlicher Weise, unter Verwendung von Hydroxylamin-0-methyl- oder -0-ethyl-ether-Hydrochlorid und den entsprechenden Aldehyden wurden hergestellt:

80. 4,5-Dihydro-2-methoximinomethyl-1-methyl-4-oxo-imidazolo[1,2-a]chinoxalin
C₁₃H₁₂N₄O₂ MG 256 Fp. > 320°C

81. 4,5-Dihydro-2-methoximinomethyl-1-methyl-7-trifluormethyl-4-oxo-imidazolo[1,2-a]chinoxalin
C₁₄H₁₁F₃N₄O₂ MG 324 Fp. 299 - 303°C

82. 4,5-Dihydro-2-ethoximinomethyl-1-methyl-imidazolo[1,2-a]chinoxalin
C₁₄H₁₄N₄O₂ MG 270 Fp. 315 - 316°C

83. 4,5-Dihydro-2-ethoximinomethyl-1-methyl-7-trifluormethyl-4-oxo-imidazolo[1,2-a]chinoxalin
C₁₅H₁₃F₃N₄O₂ MG 338 Fp. 292 - 295°C

### Herstellung von Nitrilen:

### Beispiel 84

84. 4,5-Dihydro-1-methyl-7-trifluormethyl-4-oxo-imidazolo[1,2-a]chinoxalin-2-carbonsäurenitril
1,5 g (0,005 Mol) des in Beispiel 78 beschriebenen Oxims wurden mit 20 ml Acetanhydrid 5 h zum Sieden unter Rückfluß erhitzt. Anschließend wurde der Ansatz auf Soda-Lösung gegeben, abgesaugt und das Rohprodukt mit Methylenchlorid und Methanol umgelöst.
   Man erhielt 0,7 g (50 % d. Th.)
   C₁₃H₇F₃N₃O MG 278 Fp. > 300°C

### Herstellung von Tetrazol-Verbindungen

### Beispiel 85

85. 4,5-Dihydro-1-methyl-2-(5-tetrazolyl)-7-trifluormethyl-4-oxoimidazolo[1,2-a]chinoxalin
   1,15 g (0,004 Mol) des beschriebenen Nitrils wurden mit 0,45 g Natriumazid (0,007 Mol) und 0,4 g Ammoniumchlorid in 50 ml Dimethylformamid 16 h zum Sieden unter Rückfluß erhitzt.
   Anschließend wurde der Ansatz mit Wasser versetzt, abgesaugt, der Rückstand mit Aceton gewaschen und wiederum abgesaugt.
   Man erhielt 0,5 g (37 % d. Th.)
   C₁₃H₈F₃N₇O MG 335 Fp. > 300°C

### Beispiel 86

86. 4,5-Dihydro-1-methyl-7-trifluormethyl-4-oxo-imidazolo[1,2-a]chinoxalin-2-carbonsäure-(5-tetrazolyl)amid.
   2,0 g (0,006 Mol) der nach Beispiel 41 erhaltenen Saure wurde mit 3,9 g (0,024 Mol) Carbonyldiimidazol in 30 ml Dimethylformamid 2 h bei 80°C vorbehandelt; anschließend wurde eine Lösung von 0,6 g (0,006 Mol) wasserfreiem 5-Aminotetrazol in 10 ml Dimethylformamid zugegeben und weitere 5 h unter Rühren auf 80°C erwärmt. Zur Aufarbeitung wurde abgesaugt, das Filtrat auf Eis gegeben, angesäuert, der Niederschlag abgesaugt und das so erhaltene Produkt aus Dimethylformamid mit 2 % Wasser umkristallisiert.
   Ausbeute 0,8 g (ca. 30 % d. Th.)
   C₁₄H₉F₃N₈O₂ MG 378 Fp. > 300°C

### Herstellung von Nitroverbindungen:

### Beispiel 87

### 4,5-Dihydro-1-methyl-8-nitro-4-oxo-imidazolo[1,2-a]chinoxalin-2-carbonsäure

2,5 g (0,01 Mol) der nach Beispiel 39 gewonnen 4,5-Dihydro-1-methyl-4-oxo-imidazolo[1,2-a]chinoxalin-2-carbonsäure wurden im Verlauf von 15 min portionsweise zu 25 ml Salpetersäure der Dichte 1,50 bei 0°-5°C unter Rühren zugegeben und anschließend noch 1 h nachgerührt. Anschließend wurde der Ansatz auf Eis gegeben und das ausfallende Produkt mit Wasser und Aceton gewaschen.
Ausbeute: 2,3 g (82 % d. Th.)
C₁₂H₈N₄O₅ MG 288 Fp. > 320°C

### Beispiel 88

88. 4,5-Dihydro-1-methyl-8-nitro-7-trifluormethyl-4-oxo-imidazolo[1,2-a]chinoxalin-2-carbonsäureethylester
   5 g (0,015 Mol) 4,5-Dihydro-1-methyl-7-trifluormethyl-4-oxoimidazolo[1,2-a]chinoxalin-2-carbonsäureethylester, hergestellt nach Beispiel 4, wurden in 50 ml konzentrierter Schwefelsäure gelost und bei Raumtemperatur mit 1,8 g Kaliumnitrat portionsweise versetzt. Nach 24 h Rühren bei Raumtemperatur wurde noch 2 h auf 60°C erwärmt. Zur Aufarbeitung wurde der Ansatz auf Eis gegeben und das ausgefallene Produkt abgesaugt, das anschließend noch mit Methylenchlorid gewaschen wurde.
   Ausbeute 4,1 g (73 % d. Th.)
   C₁₅H₁₁F₃N₄O₅ MG 384 Fp. 284-286°C

### Beispiel 89

### In ähnlicher Weise wurde durch Nitrierung von 4,5-Dihydro-1-methyl-7-trifluormethyl-4-oxo-imidazolo[1,2-a]chinoxalin-2-propionsäuremethylester (hergestellt gemäß Beispiel 18)

4,5-Dihydro-1-methyl-8-nitro-7-trifluormethyl-4-oxo-imidazolo[1,2-a]chinoxalin-2-propionsäuremethylester erhalten.
C₁₆H₁₃F₃N₄O₅ MG 398 Fp. 246-250°C
und aus
4,5-Dihydro-8-chlor-1-methyl-4-oxo-imidazolo[1,2-a]chinoxalin-2-propionsäureethylester (erhalten gemäß Beispiel 28)
90. 4,5-Dihydro-8-chlor-1-methyl-7-nitro-4-oxo-imidazolo[1,2-a]chinoxalin-2-propionsäureethylester erhalten.:
   C₁₆H₁₅ClN₄O₅ MG 379 Fp. 172-174°C
Reduktion/Hydrierung von Nitroverbindungen

### Beispiel 91

### 4,5-Dihydro-8-amino-1-methyl-4-oxo-imidazolo[1,2-a]chinoxalin-2-carbonsäure

1,7 g 4,5-Dihydro-1-methyl-8-nitro-4-oxo-imidazolo[1,2-a]chinoxalin-2-carbonsäure (hergestellt nach Beispiel 88) (0,055 Mol) wurden in Eisessig/Methanol als Lösungsmittel gelöst und unter Verwendung von 1,5 Pd-C-Katalysator (10 % Pd) bei Raumtemperatur und 25°C hydriert.

Nach dem üblichen Aufarbeiten wurden 11,2 g (68 % d. Th.) Aminoverbindung erhalten.
C₁₂H₁₀N₄O₃ Mg 295 Fp. > 340°C

Beispiele für Pharmazeutische Zubereitungen:

### Beispiel A

Auf einer Tablettenpresse werden in üblicher Weise Tabletten folgender Zusammensetzung gepreßt:

| | |
|---|---|
| 40 mg | 4,5-Dihydro-1-methyl-7-trifluormethyl-4-oxo-imidazolo[1,2-a]chinoxalin-2-carbonsäure |
| 120 mg | Maisstärke |
| 13,5 mg | Gelatine |
| 45 mg | Milchzucker |
| 2,25 mg | Aerosil^{R} (chemisch reine Kieselsäure in submikroskopisch feiner Verteilung) |
| 6,75 mg | Kartoffelstärke (als 6 %iger Kleister) |

### Beispiel B

In Üblicher Weise werden Dragees folgender Zusammensetzung hergestellt:

| | |
|---|---|
| 20 mg | 4,5-Dihydro-l-methyl-7-trifluormethyl-4-oxo-imidazolo[1,2-a]chinoxalin-2-carbonsäure |
| 60 mg | Kernmasse |
| 60 mg | Verzuckerungsmasse |

Die Kernmasse besteht aus 9 Teilen Maisstärke, 3 Teilen Milchzukker und 1 Teil Luviskol^{R} VA 64 (Vinylpyrrolidon-Vinylacetat-Mischopolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Beispiel C

10 g 4,5-Dihydro-1-methyl-7-trifluormethyl-4-oxo-imidazolo[1,2-a]chinoxalin-2-carbonsäure werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

## Patentansprüche

1. Imidazolo[1,2-a]chinoxalinone der Formel I, 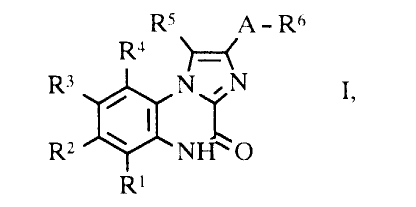 worin
A eine gesättigte oder ungesättigte Alkylengruppe mit 1-5 C-Atomen oder eine Bindung ist,
R⁶ eine Formylgruppe oder eine Carboxylgruppe, die in Form ihres Salzes mit einem physiologisch verträglichen Amin-oder Metallkation vorliegen kann, den Rest COOR₇, wobei R₇ einen C₁-C₈-Alkylrest, eine Cycloalkylgruppe mit 3-8 C-Atomen im Ring, einen Benzylrest, einen der Reste -(CH₂)ₙ-OR₈, in denen n für die Zahl 2-4 und R₈ für eine C₁-C₃-Alkylgruppe stehen, eine C₁-C₄-Hydroxyalkyl-, C₁-C₄-Alkylcarbonyl-, Nitrilo-, Tetrazolyl-, Carbonylaminotetrazol-, eine Aldoxim-, eine C₁-C₃-Alkoxyaldoxim-, oder eine gegebenenfalls substituierte Carbamoylgruppe darstellt, bedeutet,
R² ein Fluor-, Chlor- oder Bromatom, eine Trifluormethyl-, Trifluormethoxy-, Cyano-, Nitro-, Amino-, C₁-C₅-Alkyl-, C₁-C₅-Alkoxy-, Mono- oder Dialkylamino-, C₁-C₆-Alkylthio-, C₁-C₆-Alkylsulfinyl-, C₁-C₆-Alkylsulfonyl-, Aminosulfonyl-, Di-C₁-C₆-alkylaminosulfonyl- oder C₁-C₄-Alkoxycarbonylgruppe bedeutet,
R¹, R³, R⁴ die gleich oder verschieden sind und Wasserstoff-, Fluor-, Chlor- oder Bromatome, Trifluormethyl-, Trifluormethoxy-, Cyano-, Nitro-, Amino-, C₁-C₅-Alkyl-, C₁-C₅-Alkoxy-, Mono- oder Dialkylamino-, C₁-C₆-Alkylthio-, C₁-C₆-Alkylsulfinyl-, C₁-C₆-Alkylsulfonyl-, Aminosulfonyl-, Di-C₁-C₆-alkylaminosulfonyl- oder C₁-C₄-Alkoxycarbonylgruppen bedeuten, oder eines der Paare R¹,R²; R²,R³ oder R³,R⁴ auch die Gruppe -(CH₂)₄- oder -CH=CH-CH=CH- ist
R⁵ ein Wasserstoffatom, eine C₁-C₅-Alkylgruppe oder eine gegebenenfalls durch Chlor, Fluor, Trifluormethyl oder C₁₋₄-Alkyl substitutierte Phenylgruppe bedeutet,
wobei jedoch nicht gleichzeitig
A eine Bindung,
R⁶ eine Formyl-, Ethoxycarbonyl-, Hydroxymethyl-, Tetrazolyl-, Carbonsäureamid- oder 5-Carbonylaminotetrazolylgruppe,
R¹, R⁴, R⁵ Wasserstoffatome und
R² und R³ identisch und Chlor- oder Bromatome in der 7- und 8-Stellung sein können.

2. Verbindungen gemäß Anspruch 1, worin R ein Chlor- oder Bromatom, eine Trifluormethyl-, Trifluormethoxy-, oder Nitrogruppe bedeutet; R³ ein Wasserstoff- oder Chloratom oder eine Nitrogruppe ist, R⁴ ein Wasserstoff- oder Chloratom darstellt; R⁵ eine Methyl-, Ethyl- oder Phenylgruppe ist; A eine Bindung, eine Vinyl- oder Ethylengruppe darstellt und R₆ eine Carbonsäure- oder Estergruppe bedeutet.

3. Verbindungen gemäß Anspruch 1, ausgewählt aus der Gruppe bestehend aus
a. 4,5-Dihydro-7-chlor-1-methyl-4-oxc-imidazolo[1,2-a]chinoxalin-2-carbonsäure
b. 4,5-Dihydro-1-methyl-7-nitro-4-oxo-imidazolo[1,2-a]chinoxalin-2-carbonsäure
c. 4,5-Dihydro-1-methyl-7-trifluormethyl-4-oxo-imidazolo[1,2-a]chinoxalin-2-carbonsäure
d. 4,5-Dihydro-7,8-dichlor-1-methyl-4-oxo-imidazolo[1,2-a]chinoxalin-2-carbonsäure
e. 4,5-Dihydro-8-chlor-1-methyl-7-trifluormethyl-4-oxo-imidazolo[1,2-a]chinoxalin-2-carbonsäure
f. 4,5-Dihydro-8-chlor-1-isopropyl-trifluormethyl-4-oxo-imidazolo[1,2-a]chinoxalin-2-carbonsäure
g. 4,5-Dihydro-8-chlor-1-methyl-7-trifluormethyl-4-oxo-imidazolo[1,2-a]chinoxalin-2-carbonsäureethylester
h. 4,5-Dihydro-1-methyl-7-trifluormethyl-4-oxo-imidazolo[1,2-a]chinoxalin-2-carbonsäure
i. 4,5-Dihydro-1-isopropyl-7-trifluormethyl-4-oxo-imidazolo[1,2-a]chinoxalin-2-carbonsäure
j. 4,5-Dihydro-1-methyl-7-trifluormerhyl-4-oxo-imidazolo[1,2-a]chinoxalin-2-acrylsäure
k. 4,5-Dihydro-8-chlor-1-ethyl-7-trifluormethyl-4-oxo-imidazolo[1,2-a]chinoxalin-2-carbonsäuremethylester
l. 4,5-Dihydro-8-chlor-1-ethyl-7-trifluormethyl-4-oxo-imidazolo[1,2-a]chinoxalin-2-carbonsäure
m. 4,5-Dihydro-1-methyl-7-trifluormethyl-4-oxo-imidazolo[1,2-a]chinoxalin-2-propionsäure
n. 4,5-Dihydro-8-chlor-7-trifluormethyl-4-oxo-imidazolo[1,2-a]chinoxalin-2-carbonsäure
o. 4,5-Dihydro-1-phenyl-7-trifluormethyl-4-oxo-imidazolo[1,2-a]chinoxalin-2-carbonsäure-ethylester
p. 4,5-Dihydro-1-phenyl-7-trifluormethyl-4-oxo-imidazolo[1,2-a]chinoxalin-2-carbonsäure
q. 4,5-Dihydro-1-methyl-8-nitro-7-trifluormethyl-4-oxo-imidazolo[1,2-a]chinoxalin-2-carbonsäureethylester
r. 4,5-Dihydro-1-methyl-8-nitro-7-trifluormethyl-4-oxo-imidazolo[1,2-a]chinoxalin-2-carbonsäure
s. 4,5-Dihydro-1-methyl-8-nitro-7-trifluormethyl-4-oxo-imidazolo[1,2-a]chinoxalin-2-propionsäuremethylester
t. 4,5-Dihydro-1-methyl-8-nitro-7-trifluormethyl-4-oxo-imidazolo[1,2-a]chinoxalin-2-propionsäure
u. 4,5-Dihydro-1-methyl-7-nitro-4-oxo-imidazolo[1,2-a]chinoxalin-2-propionsäure
v. 4,5-Dihydro-8-chlor-1-methyl-7-trifluormethyl-4-oxo-imidazolo[1,2-a]chinoxalin-2-propionsäureethylester
w. 4,5-Dihydro-8-chlor-1-methyl-7-trifluormethyl-4-oxo-imidazolo[1,2-a]chinoxalin-2-propionsäure
x. 4,5-Dihydro-1-phenyl-7-trifuormethyl-4-oxo-imidazolo[1,2-a]chinoxalin-2-propionsäure
y. 4,5-Dihydro-8-chlor-1-methyl-7-trifluormethyl-4-oxo-imidazolo[1,2-a]chinoxalin-2-propionsäureethylester

4. Arzneimittel, enthaltend Verbindungen der Formel I oder deren therapeutisch verträgliche Salze.

5. Verbindungen der Formel I gemäß Anspruch 1 zur Verwendung bei der Bekämpfung von Krankheiten.

6. Verfahren zur Herstellung der Imidazolo[1,2-a]chinoxalinone der Formel I gemäß Anspruch 1, dadurch gekennzeichnet, daß man eine Verbindung der Formel II 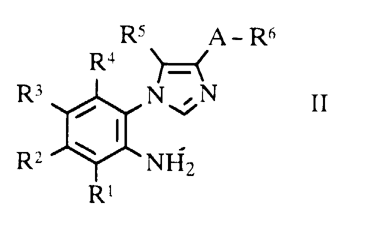 worin R¹ - R⁵ und A die angegebene Bedeutung haben und R⁶ eine Nitril-, Carbonsäureester-, Aldehyd-, oder Alkanoylgruppe bedeutet, mit einem doppelt aktivierten Kohlensäurederivat, umsetzt und
a. - falls R⁶ im Endprodukt der Formel I eine COOH-Gruppe darstellt - einen so erhaltenen Ester (R⁶= COOR⁷) verseift oder
b. - falls R⁶ im Endprodukt der Formel I eine Hydroxyalkyl-, Aldehyd-, Oxim- oder Oximethergruppe bedeutet - einen so erhaltenen Ester (R⁶= COOR⁷) reduziert und die so erhaltene Hydroxyalkylverbindung gegebenenfalls anschließend zum Aldehyd oxidiert und diesen gegebenenfalls mit Hydroxylamin bzw. Hydroxylamin-O-alkylethern zum Oxim bzw. Oximether umsetzt oder
c. - falls R⁶ im Endprodukt der Formel I eine gegebenenfalls substituierte Carbamoyl- oder Carbonylaminotetrazolgruppe bedeutet - eine gemäß a) erhaltene Säure gegebenenfalls in aktivierter Form mit Ammoniak, einem Amin oder 5-Aminotetrazol umsetzt oder
d. - falls R⁶ einen Tetrazolylrest bedeutet - eine Verbindung der Formel I mit R⁶ in der Bedeutung einer Nitrilgruppe mit Stickstoffwasserstoffsäure oder einem ihrer Salze umsetzt.
und die so erhaltenen Verbindungen gegebenenfalls nitriert und/oder in ihre physiologisch verträglichen Salze überführt.

## Claims

1. An imidazolo[1,2-a]quinoxalinone of the formula I 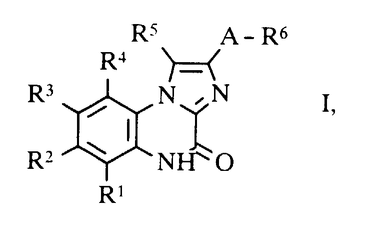 where
A is a saturated or unsaturated alkylene group with 1-5 C atoms or a bond,
R⁶ is a formyl group or a carboxyl group which can be in the form of its salt with a physiologically tolerated amine or metal cation, the radical COOR₇ where R₇ is C₁-C₈-alkyl, cycloalkyl with 3-8 C atoms in the ring, benzyl, -(CH₂)ₙ-OR₈ where n is 2-4 and R₈ is C₁-C₃-alkyl, or is C₁-C₄-hydroxyalkyl, C₁-C₄-alkylcarbonyl, cyano, tetrazolyl, tetrazolylcarbamoyl, hydroxyiminomethyl, C₁-C₃-alkoxylminomethyl or unsubstituted or substituted carbamoyl,
R² is fluorine, chlorine, bromine, trifluoromethyl, trifluoromethoxy, cyano, nitro, amino, C₁-C₅-alkyl, C₁-C₅-alkoxy, mono- or dialkylamino, C₁-C₆-alkylthio, C₁-C₆-alkylsulfinyl, C₁-C₆-alkylsulfonyl, aminosulfonyl, di-C₁-C₆-alkylaminosulfonyl or C₁-C₄-alkoxycarbonyl,
R¹, R³ and R⁴ are identical or different and are each hydrogen, fluorine, chlorine, bromine, trifluoromethyl, trifluoromethoxy, cyano, nitro, amino, C₁-C₅-alkyl, C₁-C₅-alkoxy, mono- or dialkylamino, C₁-C₆-alkylthio, C₁-C₆-alkylsulfinyl, C₁-C₆-alkylsulfonyl, aminosulfonyl, di-C₁-C₆-alkylaminosulfonyl or C₁-C₄-alkoxycarbonyl, or one of the pairs R¹ and R², R² and R³ or R³ and R⁴ is also -(CH₂)₄- or -CH=CH-CH=CH-,
R⁵ is hydrogen, C₁-C₅-alkyl or phenyl which is unsubstituted or substituted by chlorine, fluorine, trifluoromethyl or C₁₋₄-alkyl,
but where the following may not simultaneously be true
A is a bond,
R⁶ is formyl, ethoxycarbonyl, hydroxymethyl, tetrazolyl, carbamoyl or 5-tetrazolylcarbamoyl,
R¹, R⁴ and R⁵ are each hydrogen and
R² and R³ are identical and are each chlorine or bromine in positions 7 and 8.

2. A compound as claimed in claim 1, where R² is chlorine, bromine, trifluoromethyl, trifluoromethoxy or nitro; R³ is hydrogen, chlorine or nitro; R⁴ is hydrogen or chlorine; R⁵ is methyl, ethyl or phenyl; A is a bond or a vinyl or ethylene group, and R⁶ is a carboxylic acid or ester moiety.

3. A compound as claimed in claim 1, selected from the group comprising
a. 4,5-dihydro-7-chloro-1-methyl-4-oxoimidazolo[1,2-a]quinoxaline-2-carboxylic acid
b. 4,5-dihydro-1-methyl-7-nitro-4-oxoimidazolo[1,2-a]quinoxaline-2-carboxylic acid
c. 4,5-dihydro-1-methyl-7-trifluoromethyl-4-oxoimidazolo[1,2-a]quinoxaline-2-carboxylic acid
d. 4,5-dihydro-7,8-dichloro-1-methyl-4-oxoimidazolo[1,2-a]quinoxaline-2-carboxylic acid
e. 4,5-dihydro-8-chloro-1-methyl-7-trifluoromethyl-4-oxoimidazolo[1,2-a]quinoxaline-2-carboxylic acid
f. 4,5-dihydro-8-chloro-1-isopropyl-trifluoromethyl-4-oxoimidazolo[1,2-a]quinoxaline-2-carboxylic acid
g. ethyl 4,5-dihydro-8-chloro-1-methyl-7-trifluoromethyl-4-oxoimidazolo[1,2-a]quinoxaline-2-carboxylate
h. 4,5-dihydro-1-methyl-7-trifluoromethyl-4-oxoimidazolo[1,2-a]quinoxaline-2-carboxylic acid
i. 4,5-dihydro-1-isopropyl-7-trifluoromethyl-4-oxoimidazolo[1,2-a]quinoxaline-2-carboxylic acid
j. 4,5-dihydro-1-methyl-7-trifluoromethyl-4-oxoimidazolo[1,2-a]quinoxaline-2-acrylic acid
k. methyl 4,5-dihydro-8-chloro-1-ethyl-7-trifluoromethyl-4-oxoimidazolo[1,2-a]quinoxaline-2-carboxylate
l. 4,5-dihydro-8-chloro-1-ethyl-7-trifluoromethyl-4-oxoimidazolo[1,2-a]quinoxaline-2-carboxylic acid
m. 4,5-dihydro-1-methyl-7-trifluoromethyl-4-oxoimidazolo[1,2-a]quinoxaline-2-propionic acid
n. 4,5-dihydro-8-chloro-7-trifluoromethyl-4-oxoimidazolo[1,2-a]quinoxaline-2-carboxylic acid
o. ethyl 4,5-dihydro-1-phenyl-7-trifluoromethyl-4-oxoimidazolo[1,2-a]quinoxaline-2-carboxylate
p. 4,5-dihydro-1-phenyl-7-trifluoromethyl-4-oxoimidazolo[1,2-a]quinoxaline-2-carboxylic acid
q. ethyl 4,5-dihydro-1-methyl-8-nitro-7-trifluoromethyl-4-oxoimidazolo[1,2-a]quinoxaline-2-carboxylate
r. 4,5-dihydro-1-methyl-8-nitro-7-trifluoromethyl-4-oxoimidazolo[1,2-a]quinoxaline-2-carboxylic acid
s. methyl 4,5-dihydro-1-methyl-8-nitro-7-trifluoromethyl-4-oxoimidazolo[1,2-a]quinoxaline-2-propionate
t. 4,5-dihydro-1-methyl-8-nitro-7-trifluoromethyl-4-oxoimidazolo[1,2-a]quinoxaline-2-propionic acid
u. 4,5-dihydro-1-methyl-7-nitro-4-oxoimidazolo[1,2-a]quinoxaline-2-propionic acid
v. ethyl 4,5-dihydro-8-chloro-1-methyl-7-trifluoromethyl-4-oxoimidazolo[1,2-a]quinoxaline-2-propionate
w. 4,5-dihydro-8-chloro-1-methyl-7-trifluoromethyl-4-oxoimidazolo[1,2-a]quinoxaline-2-propionic acid
x. 4,5-dihydro-1-phenyl-7-trifluoromethyl-4-oxoimidazolo[1,2-a]quinoxaline-2-propionic acid
y. ethyl 4,5-dihydro-8-chloro-1-methyl-7-trifluoromethyl-4-oxoimidazolo[1,2-a]quinoxaline-2-propionate.

4. A drug containing a compound of the formula I or its therapeutically utilizable salts.

5. A compound of the formula I as claimed in claim 1 for use for controlling diseases.

6. A process for preparing imidazolo[1,2-a]quinoxalinones of the formula I as claimed in claim 1, which comprises reacting a compound of the formula II 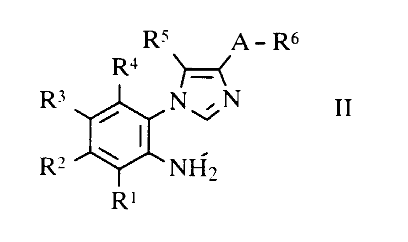 where R¹ - R⁵ and A have the stated meanings, and R⁶ is a nitrile, carboxylic ester, aldehyde or alkanoyl moiety, with a doubly activated carbonic acid derivative, and
a. if R⁶ in the final product of the formula I is COOH, hydrolyzing an ester (R⁶=COOR⁷) obtained in this way or
b. if R⁶ in the final product of the formula I is a hydroxyalkyl, aldehyde, oxime or oxime ether moiety, reducing an ester (R⁶=COOR⁷) obtained in this way and, where appropriate, subsequently oxidizing the resulting hydroxyalkyl compound to the aldehyde and reacting the latter where appropriate with hydroxylamine or hydroxylamine O-alkyl ethers to give the oxime or oxime ether, or
c. if R⁶ in the final product of the formula I is an unsubstituted or substituted carbamoyl or tetrazolylcarbamoyl group, reacting an acid obtained as in a) where appropriate in activated form with ammonia, an amine or 5-aminotetrazole or
d. if R⁶ is a tetrazolyl radical, reacting a compound of the formula I where R⁶ is a cyano group with hydrocyanic acid or one of its salts,
and, where appropriate, nitrating the compounds obtained in this way and/or converting them into their physiologically tolerated salts.

## Revendications

1. Imidazolo[1,2-a]quinoxalinones de formule I 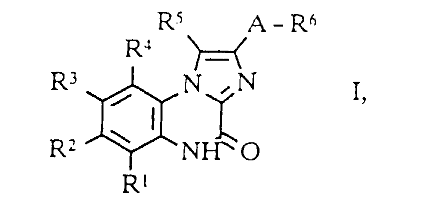 dans laquelle
A représente un groupe alkylène saturé ou insaturé en C1-C5 ou une liaison,
R⁶ représente un groupe formyle ou un groupe carboxyle qui peut être à l'état de sel d'un cation aminé ou métallique acceptable pour les usages pharmaceutiques, le groupe COOR⁷ dans lequel R⁷ représente un groupe alkyle en C1-C8, un groupe cycloalkyle contenant trois à huit atomes de carbone cycliques, un groupe benzyle, l'un des groupes -(CH₂)ₙ-OR⁸ pour lesquels n est un nombre allant de 2 à 4 et R⁸ représente un groupe alkyle en C1-C3, un groupe hydroxyalkyle en C1-C4, (alkyle en C1-C4)carbonyle, nitrilo, tétrazolyle, carbonylaminotétrazole, un groupe aldoxime, (alcoxy en C1-C3)aldoxime ou un groupe carbamoyle éventuellement substitué,
R² représente un atome de fluor, de chlore ou de brome, un groupe trifluorométhyle, trifluorométhoxy, cyano, nitro, amino, alkyle en C1-C5, alcoxy en C1-C5, mono- ou di-alkylamino, alkylthio en C1-C6, alkylsulfinyle en C1-C6, alkylsulfonyle en C1-C6, aminosulfonyle, di-(alkyle en C1-C6)aminosulfonyle ou (alcoxy en C1-C4)carbonyle,
R¹, R³, R⁴, ayant les significations identiques ou différentes, représentent chacun un atome d'hydrogène, de fluor, de chlore ou de brome, un groupe trifluorométhyle, trifluorométhoxy, cyano, nitro, amino, alkyle en C1-C5, alcoxy en C1-C5, mono- ou di-alkylamino, alkylthio en C1-C6, alkylsulfinyle en C1-C6, alkylsulfonyle en C1-C6, aminosulfonyle, di-(alkyle en C1-C6)aminosulfonyle ou (alcoxy en C1-C4)carbonyle, ou bien l'une des paires R¹, R² ; R², R³ ou R³, R⁴ représente un groupe -(CH₂)₄ ou -CH=CH-CH=CH-
R⁵ représente un atome d'hydrogène, un groupe alkyle en C1-C5 ou un groupe phényle éventuellement substitué par le chlore, le fluor, des groupes trifluorométhyle ou alkyle en C1-C4,
sous réserve que les significations suivantes, simultanées, sont exclues :
A : une liaison,
R⁶ : un groupe formyle, éthoxycarbonyle, hydroxyméthyle, tétrazolyle, carboxamide ou 5-carbonylaminotétrazolyle,
R¹, R⁴, R⁵ : des atomes d'hydrogène et
R² et R³, identiques, des atomes de chlore ou de brome en positions 7 et 8.

2. Composés selon la revendication 1, pour lesquels R² représente un atome de chlore ou de brome, un groupe trifluorométhyle, trifluorométhoxy ou nitro ; R³ représente un atome d'hydrogène ou de chlore ou un groupe nitro, R⁴ représente un atome d'hydrogène ou de chlore ; R⁵ représente un groupe méthyle, éthyle ou phényle ; A représente une liaison, un groupe vinyle ou éthylène et R⁶ un groupe acide carboxylique ou ester.

3. Composés selon la revendication 1, choisis dans le groupe consistant en :
a. l'acide 4,5-dihydro-7-chloro-1-méthyl-4-oxo-imidazolo [1,2-a]quinoxaline-2-carboxylique,
b. l'acide 4,5-dihydro-1-méthyl-7-nitro-4-oxo-imidazolo[1,2-a]quinoxaline-2-carboxylique
c. l'acide 4,5-dihydro-1-méthyl-7-trifluorométhyl-4-oxo-imidazolo[1,2-a]quinoxaline-2-carboxylique
d. l'acide 4,5-dihydro-7,8-dichloro-1-méthyl-4-oxo-imidazolo[1,2-a]quinoxaline-2-carboxylique
e. l'acide 4,5-dihydro-8-chloro-1-méthyl-7-trifluorométhyl-4-oxo-imidazolo[1,2-a]quinoxaline-2-carboxylique
f. l'acide 4,5-dihydro-8-chloro-1-isopropyl-trifluorométhyl-4-oxo-imidazolo[1,2-a]quinoxaline-2-carboxylique
g. l'acide 4,5-dihydro-8-chloro-1-méthyl-7-trifluorométhyl-4-oxo-imidazolo[1,2-a]quinoxaline-2-carboxylique
h. l'acide 4,5-dihydro-1-méthyl-7-trifluorométhyl-4-oxo-imidazolo[1,2-a]quinoxaline-2-carboxylique
i. l'acide 4,5-dihydro-1-isopropyl-7-trifluorométhyl-4-oxo-imidazolo[1,2-a]quinoxaline-2-carboxylique
j. l'acide 4,5-dihydro-1-méthyl-7-trifluorométhyl-4-oxo-imidazolo[1,2-a]quinoxaline-2-acrylique
k. le 4,5-dihydro-8-chloro-1-éthyl-7-trifluorométhyl-4-oxo-imidazolo[1,2-a]quinoxaline-2-carboxylate de méthyle
l. l'acide 4,5-dihydro-8-chloro-1-éthyl-7-trifluorométhyl-4-oxoimidazolo[1,2-a]quinoxaline-2-carboxylique
m. l'acide 4,5-dihydro-1-méthyl-7-trifluorométhyl-4-oxo-imidazolo[1,2-a]quinoxaline-2-propionique
n. l'acide 4,5-dihydro-8-chloro-7-trifluorométhyl-4-oxo-imidazolo[1,2-a]quinoxaline-2-carboxylique
o. le 4,5-dihydro-1-phényl-7-trifluorométhyl-4-oxo-imidazolo[1,2-a]quinoxaline-2-carboxylate d'éthyle
p. l'acide 4,5-dihydro-1-phényl-7-trifluorométhyl-4-oxo-imidazolo[1,2-a]quinoxaline-2-carboxylique
q. le 4,5-dihydro-1-méthyl-8-nitro-7-trifluorométhyl-4-oxo-imidazolo[1,2-a)quinoxaline-2-carboxylate d'éthyle
r. l'acide 4,5-dihydro-1-méthyl-8-nitro-7-trifluorométhyl-4-oxo-imidazolo[1,2-a]quinoxaline-2-carboxylique
s. le 4,5-dihydro-1-méthyl-8-nitro-7-trifluorométhyl-4-oxo-imidazolo[1,2-a]quinoxaline-2-propionate de méthyle
t. l'acide 4,5-dihydro-1-méthyl-8-nitro-7-trifluorométhyl-4-oxo-imidazolo[1,2-a]quinoxaline-2-propionique
u. l'acide 4,5-dihydro-1-méthyl-7-nitro-4-oxo-imidazolo[ 1,2-a]quinoxaline-2-propionique
v. le 4,5-dihydro-8-chloro-1-méthyl-7-trifluorométhyl-4-oxo-imidazolo[1,2-a]quinoxaline-2-propionate d'éthyle
w. l'acide 4,5-dihydro-8-chloro-1-méthyl-7-trifluorométhyl-4-oxo-imidazolo[1,2-a]quinoxaline-2-propionique
x. l'acide 4,5-dihydro-1-phényl-7-trifluorométhyl-4-oxo-imidazolo[1,2-a]quinoxaline-2-propionique
y. le 4,5-dihydro-8-chloro-1-méthyl-7-trifluorométhyl-4-oxo-imidazolo[1,2-a]quinoxaline-2-propionate d'éthyle.

4. Médicament contenant des composés de formule I ou leurs sels acceptables pour l'usage thérapeutique.

5. Composés de formule I selon la revendication 1 pour l'utilisation dans le traitement de maladies.

6. Procédé de préparation des imidazolo[1,2-a]quinoxalinones de formule I de la revendication 1, caractérisé par le fait que l'on fait réagir un composé de formule II 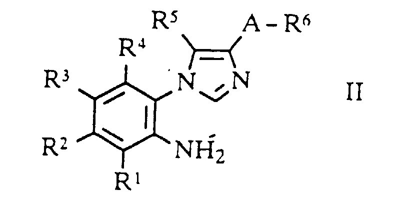 dans laquelle R¹ à R⁵ et A ont les significations indiquées ci-dessus et R⁶ représente un groupe nitrile, ester d'acide carboxylique, aldéhyde ou alcanoyle, avec un dérivé de l'acide carbonique à double activation et
a. lorsque, dans le produit final de formule I, R⁶ représente un groupe COOH, on saponifie un ester ainsi obtenu (R⁶ = COOR⁷), ou bien
b. lorsque, dans le produit final de formule I, R⁶ représente un groupe hydroxyalkyle, aldéhyde, oxime ou éther d'oxime, on réduit un ester ainsi obtenu (R⁶ = COOR⁷), ce qui donne un dérivé hydroxyalkylé qu'on oxyde ensuite le cas échéant en aldéhyde, lequel, le cas échéant, est converti en oxime ou éther d'oxime par réaction avec l'hydroxylamine ou des éthers O-alkyliques de l'hydroxylamine, ou bien
c. lorsque, dans le produit final de formule I, R⁶ représente un groupe carbamoyle éventuellement substitué ou carbonylaminotétrazole, on fait réagir un acide obtenu en a), le cas échéant sous forme activée, avec l'ammoniac, une amine ou le 5-aminotétrazole, ou bien
d. lorsque R⁶ représente un groupe tétrazolyle, on fait réagir un composé de formule I dans laquelle R⁶ représente un groupe nitrile avec l'acide azothydrique ou l'un de ses sels,
et le cas échéant, on nitre les composés ainsi obtenus et/ou on les convertit en leurs sels acceptables pour l'usage pharmaceutique.
